# EUROPEAN PATENT APPLICATION

(11) **EP 3 693 020 A1**
(43) Date of publication of application: **12.08.2020**
(21) Application number: 20152229.9
(22) Date of filing: 16.01.2020
(51) Int. Cl.: A61K 47/12, A61K 47/20, A61K 9/00, A61K 33/00, A61P 23/00, A61P 25/00

(54) **POTASSIUM ENRICHED TOPICAL FORMULATIONS FOR PAIN RELIEF AND SLEEP AID**

(30) Priority: 08.02.2019 US 201962803277 P
(71) Applicant: Burmaster International Group GmbH, 50676 Köln (DE)
(72) Inventor: Burmaster, Brian M., 50676 Köln (DE)
(74) Representative: Michalski Hüttermann & Partner Patentanwälte mbB

(57) **Abstract**

This invention describes topical formulations for pain relief and sleep aid that utilizes potassium salts, as the active ingredient. More specifically, the invention relates to a topical pain relief and sleep aid formulation comprising a potassium ion, as the active ingredient, electrically balanced by an anion, wherein said anion is selected from the FDAs GRAS list.

## Description

### FIELD OF INVENTION

This invention describes topical formulations for pain relief and sleep aid that utilizes potassium salts, as the active ingredient.

### BACKGROUND OF INVENTION

Pain relief medications, prescribed or over the counter (OTC) are varied and wide spread, as can be easily confirmed by a visit to any retail pharmacy or a nutrition center. Medication are administered through enteral (oral, sublingual, rectal) and parenteral (injection, inhalation, and topical) routes.

Severe pain usually requires injectables, as to quickly deliver the medication to the blood stream, including the pain source. A large number of common pain medications, however, are administered orally or topically. Enteral (particularly oral) delivery of medications takes a longer time to reach pain source, due to the fact that the medications must first enter the digestion system and, pending surviving its environment, then enter the blood stream.

Oral delivery of pain relief medications is widely used, as most patients view oral delivery as convenient, and due to fear of needle injection "needle phobia". However, there are drawbacks, as seen in Figure One for oral delivery: i)- medications must enter and survive the digestive system without degrading; ii)- the medications are delivered to all parts of the patient's body containing blood vessels, which can lead to undesirable and often harmful side effects to organs which are not intended to be medicated; and iii)- oral medications, in e.g., tablet form, which can cause overdosing, leading to hyperkalemia. Hyperkalemia, a potentially life-threatening condition in which serum potassium exceeds 5.5 mmol/l (millimole per liter, citing Merck Index). Severe hyperkalemia ([K+] >7.5 mmol/l) can lead to disturbances of cardiac rhythm, which can be fatal. Additionally, hyperkalemia can cause irreversible kidney disease. At extremely high levels, temporary paralysis can ensue. Drawbacks in items ii)- and iii)-, above, can also result from injectable delivery.

Topical delivery is usually slower, as the medication must permeate, or diffuse, through the patient's skin. Topical delivery, however, enhances the delivery of a larger proportion of the medication to the pain source, and thus limits harmful side effects on other organs.

There exists a need for formulations containing an active form of a potassium salt that provide potassium ions that can be applied topically on the pain area for fast pain relief, while also avoiding potassium ion overdosing (hyperkalemia). The present invention provides formulations to minimize the potentially dangerous overdose situations by supplying potassium topically to the pain area, thus decreasing the probability of the aforementioned unwanted side-effects.

Prior art: US patent 7,214,666 describes a nutritional supplement to repair articular cartridge, with the active ingredients containing combinations of chiefly complex carbohydrates such as chondroitin sulfate sodium, various salts of glucosamine, and chelated manganese proteinate. Although potassium is present in these compositions, it is not indicated that it is the active ingredient, as other cations are also present.

US patent 6,953,817 describes a two-component dental composition to reduce pain associated with dentinal hypersensitivity. The patent describes a method to produce A first dentifrice component having a neutral pH in the range of about 6.5 to 7.5 buffered with a phosphate salt, a second dentifrice component having an alkaline pH, where at least one of the components containing potassium ions, with fluoride ions. The mode of action for pain relief is explained to be due the use of potassium Salts in combination with gradual mineralization on the dentin surface which can either totally or partially occlude dentin tubules.

US patent US4564521 describes a medication for pain relief in joints of humans, where an aqueous solution of Iodine, Sodium iodine or potassium iodine, Ethanol alcohol, and Lemon extract. It is not indicated that potassium ions play an active role, since it is replaceable by sodium ions, to produce same outcome.

Since potassium salts are common reagents, they are present in many compounds and formulations. They are usually present as counter ions in oral pills, and even as a potassium oral potassium supplements, such as the Potassium 99 Plus, detailed below. Another example is Diclofenac (common brand names: Cataflam, Zipsor), with the generic name of Diclofenac Potassium. In this compound, is deduced to be is the Diclofenac moiety, as sodium salt of Diclofenac also is used for the same purpose, as an anti-inflammatory.

### SUMMARY OF THE INVENTION

This application describes compositions and applications of topical potassium salt compositions, for the purpose of being used as a pain-reliever, particularly for muscle cramps and sleep aid. The compositions provide convenient and localized rapid relief. Besides convenience, the compositions can be formulated to possess fast and controllable release of active components.

### Detailed Description

This application describes compositions and applications of topical potassium salt compositions, for the purpose of being used as a topical pain reliever. The compositions provide convenient and localized rapid relief. Besides convenience, the compositions can be formulated to possess fast and controllable release of active components.

A major objective of the present application is muscular pain relief, particularly after strenuous exercise. For example, it is common that marathon runners suffer post-race running cramps. A relief of the cramps is suggested to be gained by taking potassium-enriched foods, such as bananas or supplements such as Potassium 99 Plus, sold in General Nutrition Stores, as shown in Figure Two. This has been the experience of the inventor Burmaster, who has completed 64 marathons.

Potassium-containing pills and potassium-enriched foods, such as bananas are usually taken during or after the race. Orally delivered treatments suffer from drawbacks discussed above, including usually requiring higher levels to be introduced to the entire cardio- vascular system to areas within the body that may not require potassium. To supply a needed amount of the medication to the pain site, a higher dose is administered, which can lead to (potassium) overdose, causing undesirable complications discussed above.

Cramp pains are attributed to various causes, such as dehydration, loss of water (dehydration) and electrolytes (sodium and/ or potassium salts, see Stofan, J.R. et. al., Sweat and Sodium Losses in NCAA Football Players: A Precursor to Heat Cramps? in "International Journal of Sport Nutrition and Exercise Metabolism", 2005, 15, 641-652) or running speed/history of cramps (see: Schwellnus, M. "Increased running speed and previous cramps rather than dehydration or serum sodium changes predict exercise-associated muscle cramping: A prospective cohort study in 210 Ironman triathletes", in British Journal of Sports Medicine 45(8):650-6, 2010).

Without being adherent to any particular theory of cramp relief mechanism, this application provides a practical formulation to provide substantially faster pain relief which has been observed by the inventor (Burmaster).

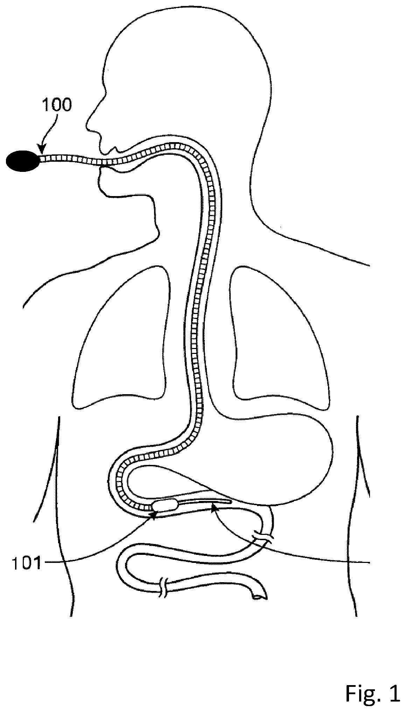

Unlike commercially available potassium salt tablets, which are taken orally and digested before entering into the blood stream, as seen in Figure 1 whereby the pill (item#100) follows the digestion tract before becoming dissolved as (item#101) and then finally distributed throughout the blood circulation system, with a small fraction of the original active component portioned to the pain site.

Potassium salts in tablet form are commercially available, for example, at General Nutrition Center (GNC), as shown in Figure Two of the GNC Potassium 99 Plus Tablets with the stated Ingredients.

### PREFERRED EMBODIMENTS

An important aspect of this invention is to provide an effective, safe, and user-friendly formulation for topical pain relief.

Another important aspect of this invention is to include a safe level of potassium ions directly to the pain site. The pain site being a muscular part of the body, suffering from pain or cramps as a result of strenuous exercise and excessive physical efforts or other causes.

Note: The total amount of circulating potassium in blood (approximately 5 liters in average adult humans) is between and 17.5 mmol and 25.5 mmol. It must also be taken into account the partitioning of K+ between the composition and the patient body, which depends on the permeability of the skin to K+. The permeability is a function of skin type, moisture level, and type of skin. It is therefore expected that only a small fraction of K+ would migrate from the composition into the skin.

The selection of the K+ counter ion (anion) can be one of any anions derived from the United States' Food and Drug Administration (FDA), substances that are Generally Recognized As Safe (GRAS) list. The GRAS substances are included in Table I, and are listed on the FDA web site and found in the FDA booklet under the title "PART 182-SUBSTANCES GENERALLY RECOGNIZED AS SAFE" and found in Code of Federal Regulations §182.1+

By the counter ion (anion) can be one of any anions derived from the GRAS list, it is meant, for example, when selecting glutamate acid (item §182.1045 in Table I), the (derived) anion is the glutamate ion. Note that this apply only to ionic substances that can be dissociated into positively and negatively charged constituents.

**Table 1: Substances Generally Recognized as Safe (GRAS)**

| Subpart B-Multiple Purpose GRAS Food Substances | |
|---|---|
| | Glutamic acid. |
| §182.1047 | Glutamic acid hydrochloride. |
| §182.1057 | Hydrochloric acid. |
| §182.1073 | Phosphoric acid. |
| §182.1087 | Sodium acid pyrophosphate. |
| §182.1125 | Aluminum sulfate. |
| §182.1127 | Aluminum ammonium sulfate. |
| §182.1129 | Aluminum potassium sulfate. |
| §182.1131 | Aluminum sodium sulfate. |
| §182.1180 | Caffeine. |
| §182.1217 | Calcium phosphate. |
| §182.1235 | Caramel. |
| §182.1320 | Glycerin. |
| §182.1480 | Methylcellulose. |
| §182.1500 | Monoammonium glutamate. |
| §182.1516 | Monopotassium glutamate. |
| §182.1711 | Silica aerogel. |
| §182.1745 | Sodium carboxymethylcellulose. |
| §182.1748 | Sodium caseinate. |
| §182.1778 | Sodium phosphate. |
| §182.1781 | Sodium aluminum phosphate. |
| §182.1810 | Sodium tripolyphosphate. |
| Subpart C-Anticaking Agents | |
| §182.2122 | Aluminum calcium silicate. |
| §182.2227 | Calcium silicate. |
| §182.2437 | Magnesium silicate. |
| §182.2727 | Sodium aluminosilicate. |
| §182.2729 | Sodium calcium aluminosilicate, hydrated. |
| §182.2906 | Tricalcium silicate. |
| Subpart D-Chemical Preservatives | |
| §182.3013 | Ascorbic acid. |
| §182.3041 | Erythorbic acid. |
| §182.3089 | Sorbic acid. |
| §182.3109 | Thiodipropionic acid. |
| §182.3149 | Ascorbyl palmitate. |
| §182.3169 | Butylated hydroxyanisole. |
| §182.3173 | Butylated hydroxytoluene. |
| §182.3189 | Calcium ascorbate. |
| §182.3225 | Calcium sorbate. |
| §182.3280 | Dilauryl thiodipropionate. |
| §182.3616 | Potassium bisulfite. |
| §182.3637 | Potassium metabisulfite. |
| §182.3640 | Potassium sorbate. |
| §182.3731 | Sodium ascorbate. |
| §182.3739 | Sodium bisulfite. |
| §182.3766 | Sodium metabisulfite. |
| §182.3795 | Sodium sorbate. |
| §182.3798 | Sodium sulfite. |
| §182.3890 | Tocopherols. |
| Subpart E-Emulsifying Agents [Reserved] | |
| Subpart F-Dietary Supplements [Reserved] | |
| Subpart G-Sequestrants | |
| §182.6085 | Sodium acid phosphate. |
| §182.6197 | Calcium diacetate. |
| §182.6203 | Calcium hexametaphosphate. |
| §182.6215 | Monobasic calcium phosphate. |
| §182.6285 | Dipotassium phosphate. |
| §182.6290 | Disodium phosphate. |
| §182.6757 | Sodium gluconate. |
| §182.6760 | Sodium hexametaphosphate. |
| §182.6769 | Sodium metaphosphate. |
| §182.6778 | Sodium phosphate. |
| §182.6787 | Sodium pyrophosphate. |
| §182.6789 | Tetra sodium pyrophosphate. |
| §182.6810 | Sodium tripolyphosphate. |
| Subpart H | Stabilizers |
| §182.7255 | Chondral extract. |
| Subpart I | Nutrients |
| §182.8013 | Ascorbic acid. |
| §182.8159 | Biotin. |
| §182.8217 | Calcium phosphate. |
| §182.8223 | Calcium pyrophosphate. |
| §182.8250 | Choline bitartrate. |
| §182.8252 | Choline chloride. |
| §182.8778 | Sodium phosphate. |
| §182.8890 | Tocopherols. |
| §182.8892 | α-Tocopherol acetate. |
| §182.8985 | Zinc chloride. |
| §182.8988 | Zinc gluconate. |
| §182.8991 | Zinc oxide. |
| §182.8994 | Zinc stearate. |
| §182.8997 | Zinc sulfate. |

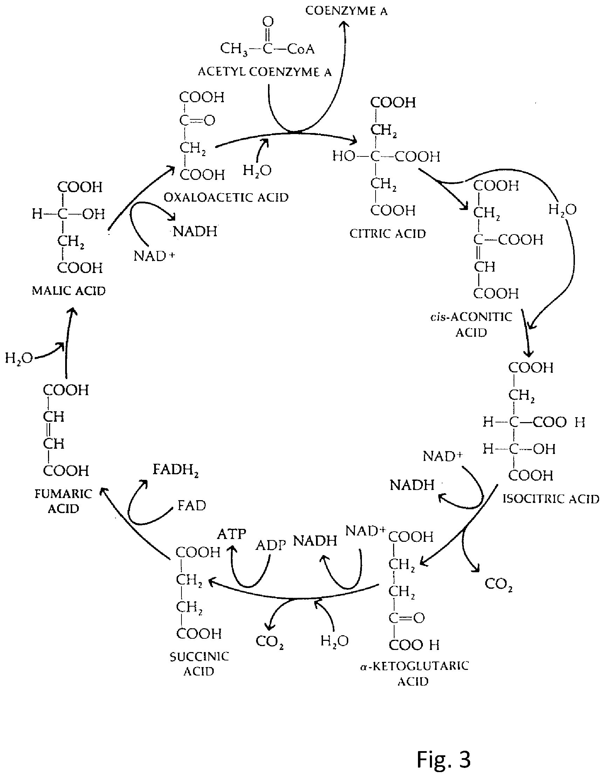

It is preferred that the counter anion is phosphate, citrate, acetate, chloride, carbonate and bicarbonate. Any imbalance between the acid-base character of the K+ and the counter anion can be compensated by adjusting the pH of the composition to nearly neutral pH. Note that K+ is a stable cation which can be precipitated or chelated by only a very small and specific group of anions/ chelators. One factor that needs to be considered is an aqueous system, the salt is totally disassociated, meaning that the anion and potassium are completely ionized and exist as separate chemical entities. In an organic system, disassociation is not 100%, meaning that the salt may exist. Since it is potassium that is the active ingredient, the best anion is one that disappears, such as the citrate ion which is consumed in the Krebs Cycle, as shown in Figure 3, leaving the potassium ion. This is also the experience of the inventor that the potassium citrate is the preferred salt, since it is fast-acting.

In a preferred and simplest embodiment, a surfactant is added to the composition, that may enhance the permeation of K+ into the skin. Ionic and neutral surfactants may be used. Example of ionic surfactant include sodium lauryl sulfate (SLS) and sodium dodecyl sulfate (SDS). Examples of neutral surfactants include Polyethylene glycol sorbitan monolaurate (Tween 20) and the various kinds of Polyethylene glycol hexadecyl ethers (Brij), e.g., Brij® 58, Brij® L4

In another embodiment, a composition that contains an active ingredient of a potassium salt, wherein the salt is contained in paste.

Another preferred embodiment is a composition that contains an active ingredient of a potassium salt, wherein the salt is contained in a cream.

Another preferred embodiment is a composition that contains an active ingredient of a potassium salt, wherein the salt is contained in an ointment.

### DESCRIPTION OF FIGURES

- Figure 1:: Oral Digestion of a Tablet
- Figure 2:: General Nutrition Center (GNC) Potassium 99+
- Figure 3:: Krebs' Citrate Cycle

### Example 1:

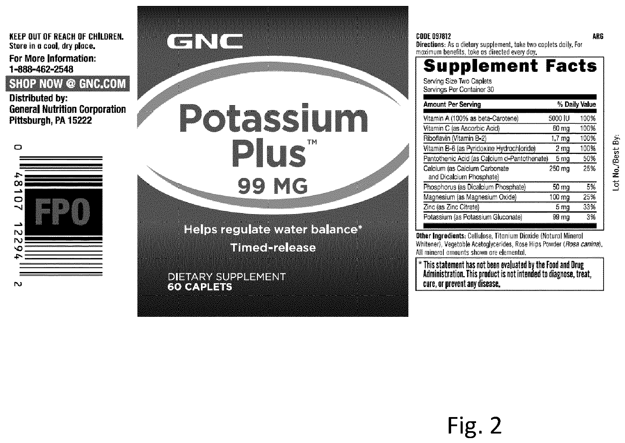

A formulation for making a colloidal formulation was prepared according to the following steps using General Nutrition Center (GNC) Potassium 99 Plus tablet described in Figure 2:
a. Grind a single potassium salt tablet (0.55 grams) into very small pieces.
b. To the crushed potassium salt add 60 cc of deionized water.
c. Bring the salt solution to boil until almost all the water has evaporated, forming a paste-like semi-solid.
d. Add the hot potassium salt paste to approximately 30 cc of a hand soap (commercially available as Sedal Yuya hand soap).
e. Stir and shake until a uniform colloidal solution is formed.
f. After cooling, apply to pain area.

This formulation was applied to leg calf cramps experienced by the inventor (Burmaster). The cramps were exacerbated because of using new, uncomfortable running shoes. After spreading 4-5 cubic centimeters (cc) of the formulation on the sore leg calves, the cramp pain was substantially reduced in 10 minutes.

The amount of potassium in the treatment portion (5 cc), of this example, is approximately 1/6 of the total volume (30 cc), which is 1/6 of one tablet (49.5 mg) of GNC's Potassium 99 Plus, or 49.5/6 mg, or 8.25 mg, which is equivalent to 0.00021 mmol. This is a small amount of K+, and well below the dangerous potassium level, so the danger of a potassium overdose is greatly reduced. This is due to the targeted delivery to the pain site.

Treatments using this formulation was applied on three different occasions (three different non-contiguous days) with similar results.

### Example 2:

The above formulation was also applied to pain in the shoulders and upper neck experienced by the inventor (Burmaster). The pain was attributable to carrying a heavy backpack to and from the gymnasium. Approximately 5 cc of the potassium salt solution was spread on the pain area, which consisted of the upper spine. This potassium formulation induced a sleep. Upon wakening (30 minutes later) the pain was substantially eliminated.

### Example 3:

The above formulation was also applied to the back of the heel, which was experiencing the onset of plantar fasciitis caused by new running shoes. Approximately 4-5 cc of the potassium salt solution were applied to the heel. After ∼10 minutes, the soreness and pain were substantially eliminated.

### Example 4:

A formulation of a topical potassium salt solution was prepared as follows: The contents of a potassium citrate capsule (product obtained from Now Foods: Corporate offices at 244 Knollwood Drive, Bloomingdale IL 60108 USA) containing 99 mg potassium citrate (inactive ingredients including silicon dioxide, stearic acid, cellulose) were mixed with 30 milliliters (ml) of Aqua Terre shampoo container, having main ingredients of: sodium lauryl sulfate (common soap), deionized water, coconut diethanolamine, amide-propyl betaine, citric acid, fragrance and sodium chloride, disodium EDTA-ZNA. After mixing, the crystals of potassium citrate dissolved except for a white insoluble silicon dioxide, which settled to the bottom.

### Example 5:

The formulation from Example 4 was applied in the amount of 4 cc to the back of the inventor (Burmaster) legs to relieve nighttime cramps and pain from an intensive workout. After 10 minutes, the pain subsided.

### Example 6:

The formulation from Example 4 was applied in the amount of 4 cc to the lower back of inventor (Burmaster) after suffering muscle spasms from lifting heavy weights at the gym. Pain substantially subsided within 10 minutes.

### Example 7:

The inventor (Brian Burmaster) was suffering from sleeplessness. This new formulation of a topical potassium citrate-99 and Sodium Lauryl Sulfate solution was applied in the amount of 2 cc to the back of my neck and the inventor was able to achieve instant and uninterrupted sleep.

## Claims

1. A topical pain relief and sleep aid formulation comprising a potassium ion, as the active ingredient, electrically balanced by an anion, wherein said anion is selected from the FDAs GRAS list.

2. The formulation in claim 1, wherein said anion is one of halide, phosphate, ascorbate, citrate, sorbate, carbonate, bicarbonate, acetate, sulfate, and tartarate.

3. The formulation in claim 1, wherein the formulation pH is adjusted to be substantially neutral.

4. The formulation in claim 3, wherein the formulation pH is adjusted to be to a pH between pH=6 and pH=8.

5. The formulation in claim 1, where the preferred anion is citrate, since it is consumed in the Kreb's cycle.

6. The formulation in claim 1 where the potassium salt is dissolved in the preferred formulation that being the surfactant sodium lauryl sulfate.

7. The formulation containing the active ingredient of a potassium salt dispersed in a surfactant applied topically for the treatment of pain, muscle cramps and sleeping problems, such as insomnia.
